# EUROPÄISCHE PATENTSCHRIFT

(11) **EP 2 774 634 B1**
(45) Veröffentlichungstag und Bekanntmachung des Hinweises auf die Patenterteilung: **02.03.2016**
(21) Anmeldenummer: 14158126.4
(22) Anmeldetag: 06.03.2014
(51) Int. Cl.: A61M 1/14, A61M 1/16, A61M 1/34, A61M 1/36

(54) **Dialysemaschine mit selbsttragendem Maschinengehäuse**
Dialysis machine with a self-supporting machine housing
Machine de dialyse avec boîtier de machine autoporteur

(30) Priorität: 07.03.2013 DE 102013102281
(43) Veröffentlichungstag der Anmeldung: 10.09.2014
(73) Patentinhaber: B. Braun Avitum AG, 34212 Melsungen (DE)
(72) Erfinder: Schäfer, Oliver, 36286 Neuenstein (DE); Ritter, Kai-Uwe, 91126 Rednitzhembach (DE)
(74) Vertreter: Winter, Brandl, Fürniss, Hübner, Röss, Kaiser, Polte - Partnerschaft mbB

(56) Entgegenhaltungen:
- CH-A1- 704 193
- US-A- 6 143 181
- US-A1- 2010 300 945

## Beschreibung

Die vorliegende Erfindung betrifft ein medizinisches Gerät zur extrakorporalen Blutbehandlung und insbesondere eine verfahrbare bzw. mobile Dialysemaschine mit einem rahmenlosen, selbsttragenden Maschinengehäuse.

### Hintergrund der Erfindung

Medizinische Geräte zur extrakoporalen Blutbehandlung, insbesondere Dialysemaschinen sind häufig als mobile Einheiten ausgebildet, welche an räumlich unterschiedliche Standorte beispielsweise innerhalb eines Krankenhauses oder eines Dialysezenfrums verfahren werden können. Auch sind Geräte dieser Gattung regelmäßigen Wartungszyklen unterzogen, welche die einwandfreie Funktionstüchtigkeit gewährleisten sollen, wobei die Wartungsarbeiten nicht auf Station sondern in hierfür speziell aus-/eingerichteten Werkstätten erfolgt, zu denen die Geräte gebracht werden müssen.

Zu diesem Zweck sind Dialysemaschinen mit einem eigenen Fahrwerk beispielsweise in Form von gelenkig gelagerten Rollen ausgerüstet, die sich an der Bodenseite des Maschinengehäuses befinden. Alternativ hierzu kann es aber auch vorgesehen sein, dass Dialysemaschinen ohne eigenes (integriertes) Fahrwerk ausgebildet sind, wobei in diesem Fall die Dialysemaschine mittels eines externen, separaten Transportmittels, beispielsweise ein Rollwagen, etc. bewegt werden müssen.

In allen Fällen einer mobilen Ausgestaltung der Dialysemaschine sind diese einer größeren mechanischen Belastung ausgesetzt, denen sie auch über einen längeren Zeitraum standhalten müssen. Insbesondere werden die Dialysemaschinen durch Personal am Maschinengehäuse hin- und herbewegt, wobei auch Erschütterungen und Vibrationen in das Gehäuse und die darin befindlichen Geräteteile übertragen werden. Darüber hinaus müssen derartige Dialysemaschinen Zugangsmöglichkeiten zu deren inneren Maschinenbestandteilen bereitstellen, über die beispielsweise Service- oder Wartungsarbeiten ausgeführt werden können.

### Stand der Technik

Medizinische Geräte zur extrakoporalen Blutbehandlung beispielsweise Dialysemaschinen, wie sie allgemein aus dem Stand der Technik bekannt sind, beinhalten eine Vielzahl unterschiedlicher Bauteile aus dem Gebiet der Elektronik, Sensorik, Hydraulik und Mechanik. Es existiert ein hydraulisches Pumpen-Filtrationssystem (Dialysierflüssigkeits-Leitungssystem) mit einer Vielzahl von Hydraulikelementen wie Pumpen, Ventilen, Filtern etc., Verrohrungen, Behältern und dergleichen, sowie einer elektronischen Steuerung mit entsprechender Sensorik, welche den Blutbehandlungsbetrieb steuert und das Gerät auf seine optimale Leistung einstellt. Diese Bauteile sind teilweise als Präzisionsbauteile ausgestaltet und daher teuer in der Anschaffung und in der Wartung. Andererseits werden teilweise einfache Bauteile, beispielsweise einzelne Verschlauchungen, insbesondere für jene Geräteabschnitte verwendet, welche als Einwegartikel vorgesehen sind.

Insgesamt stellen die verbauten Gerätebauteile einen erheblichen Kostenfaktor dar, der aufgrund der sensiblen Technologie und der erforderlichen Robustheit des Geräts durch den Einsatz preisgünstigerer Bauteile nicht besonders verringert werden kann und darf. Ein weiterer Kostenfaktor betrifft die Montage des medizinischen Geräts dieser Gattung. Grundsätzlich gilt hier, dass je weniger Montageschritte erforderlich sind, desto geringer sind die Fertigungskosten. Insoweit werden die Gesamtkosten für eine Dialysemaschine gemäß dem Stand der Technik im Wesentlichen durch die beiden vorstehend genannten Kostenblöcke bestimmt, sodass eine zumindest den einen Kostenblock betreffende Verbesserung erhebliche Auswirkungen auf die Gesamtkosten haben kann.

### Kurzbeschreibung der Erfindung

Angesichts dessen ist es die Aufgabe der vorliegenden Anmeldung, ein medizinisches Gerät zur extrakorporalen Blutbehandlung, insbesondere eine Dialysemaschine, bereit zu stellen, das mit weniger Fertigungsschritten herstellbar ist und somit kostengünstiger produziert werden kann.

Diese Aufgabe wird durch ein medizinisches Gerät zur extrakorporalen Blutbehandlung, insbesondere Dialysemaschine mit den Merkmalen des Patentanspruchs 1 gelöst. Vorteilhafte Ausgestaltungen der Erfindung sind Gegenstand der Unteransprüche.

Der Erfindung liegt die nachfolgende erfindungsgemäße Überlegung zugrunde:
Grundsätzlich ist es bekannt, medizinische Geräte zur extrakoporalen Blutbehandlung mit einem Maschinengehäuse auszustatten, das über eine tragende Rahmen- oder Gitterstruktur verfügt, welche die gesamten statischen (und auch dynamischen) Lasten aufnimmt, welche auf das Gerät einwirken. Die Gehäuseverkleidungen wie Beplankungs-/Verschalungsplatten und dergleichen Gehäusebauteile dienen zumeist nur als dekorative Hülle (Staub-/ Sichtschutz) oder stellen Öffnungsklappen oder Türen dar, welche beispielsweise Service- und Wartungszugänge temporär verschließen und die an der Rahmenstruktur vorzugsweise anscharniert sind.

Beispielsweise offenbart die US 2010 / 0 300 945 A1 ein mobiles, medizinisches Gerät zur extrakorporalen Behandlung, mit der Möglichkeit, dass das Gerät bei Leckage in einen Sicherheitsmodus umschaltet. Das System weist dabei eine Trägerstruktur, eine Gehäusekammer und zumindest eine Flüssigkeitssammelzone im Inneren auf. Die Trägerstruktur weist mindestens eine Seitenwand zur Begrenzung auf. Aus der US 2010 / 0 300 945 A1 geht lediglich hervor, dass die Trägerstruktur wenigstens die eine Seitenwand aufweist, welche also auch eine tragende Funktion übernimmt und nicht nur zur Verkleidung/Verblendung eines Rahmens/Fachwerks dient. Es wird aber nicht angegeben, dass die Trägerstruktur durch die wenigstens eine Seitenwand gebildet wird

In der Regel werden alle derartigen Gehäuse bildenden Bauteile von der Rahmenstruktur aufgenommen und von dieser in Position gehalten. Die Bauteile haben daher nur sich selbst und ggf. kleinere / leichte Anbauteile wie z.B. Elektronikleiterplatten zu tragen. Die innere Statik des Maschinengehäuses wird indessen ausschließlich durch den Gehäuserahmen bestimmt.

Ein solcher Gehäuserahmen besteht normaler Weise aus einer Mehrzahl von miteinander verschweißten, verschraubten und/oder vernieteten Trägerprofilen beispielsweise in L-, U- oder T-Form, die nach dem Prinzip des Kräftedreiecks miteinander gekoppelt sind und so die zur Aufnahme äußerer Kräfte notwendige Gehäusesteifigkeit erzeugen. Obgleich dadurch ein solches Maschinengehäuse die für den täglichen Gebrauch aus Sicht des Anwenders notwendige Robustheit aufweist, ist dessen Herstellung aufwändig und damit auch teuer. Es muss nämlich zuerst der Gehäuserahmen montiert/gefertigt werden, der dann mit den entsprechenden Gehäusebauteilen verkleidet/verschalt wird.

Die vorliegende Erfindung hat nunmehr erkannt, dass eine Vereinfachung des Maschinengehäuses technisch umsetzbar ist, ohne dass in den funktionalen Aufbau des Geräts bezüglich der verbauten Hydraulik und Elektronik eingegriffen werden muss und dabei die Zahl der Montageschritte verringert werden kann. Dies hat, wie vorstehend bereits ausgeführt wurde, einen nicht unerheblichen Einfluss auf die Fertigungskosten als einen der beiden größeren Kostenblöcke.

Inspiriert ist die vorliegende Erfindung durch die Chassiskonstruktion im Fahrzeugbau, insbesondere Automobil- und Flugzeugbau, wonach schon seit einiger Zeit vom Prinzip des Leiterrahmen abgewichen wird und statt dessen mit sogenannten selbsttragenden Karosserien gearbeitet wird. Diese Technologie zeigt, dass Fahrzeugkarosserien mit hoher Steifigkeit durch eine solche Karosserieblech - Anordnung erhaltbar sind, welche den zu erwartenden äußeren und inneren Kräften im Wesentlichen folgt. Dieses Konstruktionsprinzip wird nunmehr erfindungsgemäß auf die Gehäusekonstruktion für die Dialysemaschine übertragen.

Demnach ist es gemäß einem ersten Aspekt der vorliegende Erfindung vorgesehen, ein vorzugsweise mobiles medizinisches Gerät zur extrakoporalen Blutbehandlung mit einem im Wesentlichen rahmenlosen Geräte-/ Maschinengehäuse der selbsttragenden Bauart auszustatten. Dieses besteht vorzugsweise aus einer Anzahl von verschweißten, verschraubten, vernieteten und/oder verklebten Gehäuseplatten, die so miteinander verbunden sind, dass diese infolge äußerer, darauf einwirkender Kräfte gemäß dem normal zu erwartenden Gerätebetrieb im Wesentlichen nur schubbelastet sind/werden. Dadurch erhält das selbsttragende Gehäuse eine Steifigkeit vergleichbar zu einem Gehäuserahmen bekannter konstruktion.

Gemäß einem weiteren, ggf. unabhängigen, optionalen Aspekt der vorliegenden Erfindung sind die fest miteinander verbundenen Gehäuseplatten so angeordnet, dass das Gehäuse an vorbestimmten Stellen Ausbrüche oder Öffnungen erhält, ohne dass dadurch der Kräfteverlauf bzw. die Kräfteweiterleitung durch die Gehäuseplatten (wesentlich) unterbrochen oder beeinträchtigt wird. D.h. die verbleibenden, fest miteinander verbundenen Gehäuseplatten sind so zueinander ausgerichtet und platziert, dass das Grundprinzip der Schubbelastung der Gehäuseplatten weitestgehend aufrechterhalten bleibt und somit die Gesamtsteifigkeit des Gehäuses durch die Öffnungen geringst-möglich geschwächt/beeinträchtigt wird.

Gemäß einem weiteren, ggf. unabhängigen, optionalen Aspekt der Erfindung sind ausgewählte Gehäuseplatten partiell verstärkt, etwa um Anlenkstellen für die Öffnungen verschließende Türen oder Klappen zu bilden oder um solche Abschnitte zu versteifen, die voraussichtlich mit Kräften unterschiedlich zur Schubbelastungsrichtung der betreffenden Gehäuseplatte beaufschlagt werden (könnten). Vorzugsweise erhalten zumindest ausgewählte Verstärkungen dieser Art eine Gestalt/Struktur, die sie zur Übernahme weiterer Funktionen wie beispielsweise die Funktion als Aufnahmegehäuse von Ventilatoren oder Lüftern, als Anschusssockel für externe/adaptive Zusatzgeräte und dergleichen Funktionsträger geeignet machen.

### Figurenbeschreibung

Die Erfindung wird nachstehend anhand eines bevorzugten Ausführungsbeispiels unter Bezugnahme auf die begleitenden Figuren näher erläutert.
Fig. 1 zeigt in Prinzip- bzw. Funktionsdarstellung die eine Gehäuseseite eines medizinischen Geräts zur extrakorporalen Blutbehandlung insbesondere Dialysemaschine gemäß einem bevorzugten Ausführungsbeispiel der vorliegenden Erfindung,
Fig. 2 und 3 zeigen das Gerätegehäuse gemäß Fig. 1 in Perspektivenansicht bei geöffneten Vorder- und Hintertüren,
Fig. 4 zeigt in Perspektivenansicht die bereits montierten Seitenteile und Haube des Gerätegehäuses gemäß der Fig. 1,
Fig. 5 zeigt einen unteren Abschnitt der Vorderseite des Gerätegehäuses unterhalb der Vordertür gemäß der Fig. 2 oder 3,
Fig. 6 zeigt den zu erwartenden Schubkraftverlauf in einem Seitenteil der Gerätegehäuses gemäß der vorliegenden Erfindung,
Fig. 7 zeigt den Querschnitt eines Seitenteils mit integrierten partiellen Aussteifungen (Trägerprofilen) zur Aufnahme von zu erwartenden Quer-/Biegekräften,
Fig. 8 zeigt den zu erwartenden Kraftverlauf in der Gehäusehaube und
Fig. 9 zeigt den zu erwartenden Kraftverlauf in der Vorder- und/oder Hintertür des erfindungsgemäßen Gerätegehäuses im Fall eines geschlossenen Zustands.

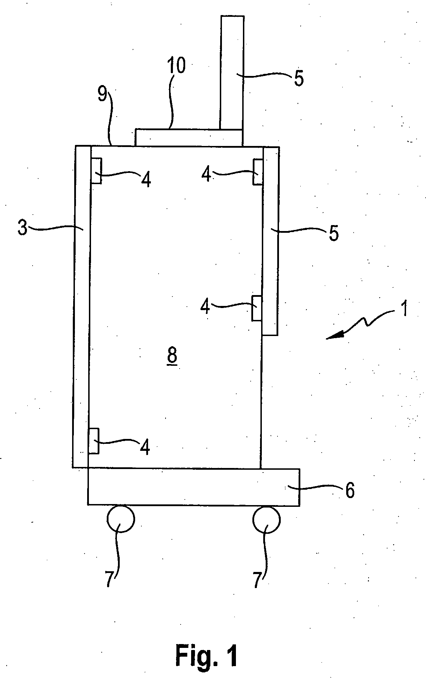

Gemäß der Fig. 1 hat das medizinische Gerät zur extrakorporalen Blutbehandlung, insbesondere Dialysemaschine, gemäß dem bevorzugten Ausführungsbeispiel der Erfindung ein Geräte- bzw. Maschinengehäuse 1, in welchem eine Anzahl von Maschinenteilen oder Maschinenkomponenten eingehaust sind oder werden können, wie beispielsweise eine Steuerelektronik, zumindest Teile der Hydraulik, Pumpen, Heizeinrichtungen und ggf. Tanks oder Beutel für ausgewählte Betriebsstoffe, welche sämtlich aus dem Stand der Technik hinlänglich bekannt sind und daher in den anliegenden Figuren nicht weiter dargestellt werden.

Das Maschinengehäuse 1 gemäß der Fig. 1 besteht im Wesentlichen aus zumindest zwei (einteiligen) Seitenplatten 8, einer zumindest teilweise als schwenkbare Tür/Klappe ausgebildeten Rückwand 3, einer Haube 9, welche die beiden (parallel beabstandeten) Seitenplatten 8 topseitig verbindet, einer Bodenplatte (oder Gerätesockel) 6, welche die beiden Seitenplatten 8 bodenseitig verbindet und einer zumindest teilweise als schwenkbare Tür/Klappe 2 ausgebildeten Vorderwand.

Die Haube 9 ist zentral mit einem Anschlusssockel 10 ausgeformt oder ausgerüstet, an welchem ein zusätzliches Gerät wie beispielsweise ein Monitor 5 montierbar/montiert ist. Die Bodenplatte 6 weist an drei oder vier in Gehäuseumfangsrichtung beabstandeten Positionen Anlenkstellen auf, an denen Geräterollen 7 montierbar/montiert sind, welche zusammen mit der Bodenplatte 6 ein Geräte - internes (integriertes) Fahrwerk bilden. Es besteht aber auch die Möglichkeit, die Bodenplatte 6 auf einem separaten Fahrwerk beispielsweise einem Rollenwagen zu platzieren.

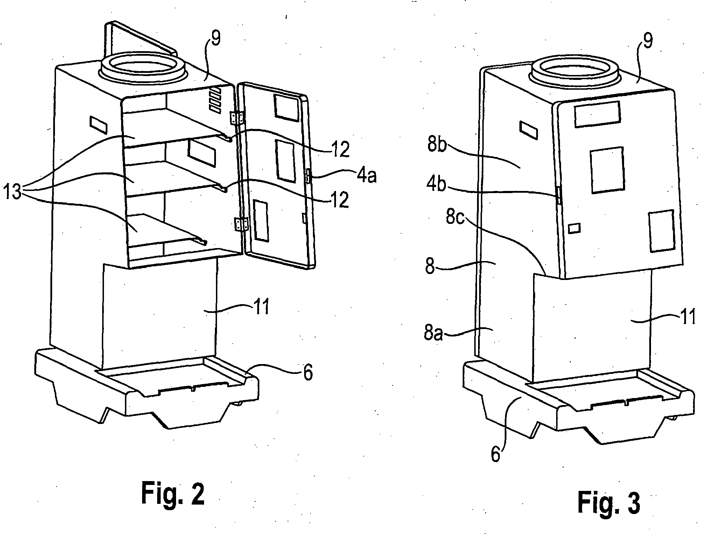

In den Fig. 2 und 3 ist das erfindungsgemäße Maschinengehäuse 1 als Konstruktionsdarstellung in Perspektivenansicht gezeigt, wobei gemäß der Fig. 2 die Gehäusetüren/-klappen 2, 3 in geöffneter und gemäß der Fig. 3 in geschlossener Position dargestellt sind.

Demzufolge sind die beiden vorzugsweise einteiligen sowie parallel beabstandeten Seitenplatten oder Seitenteile 8 in zwei höhenbeabstandete Platten abschnitte 8a, 8b theoretisch untergliederbar, nämlich einem unteren Abschnitt 8a mit geringer Tiefe und einem oberen Abschnitt 8b mit großer Tiefe, wodurch sich zwischen dem unteren und oberen Abschnitt ein Überhang 8c ergibt. Im Bereich des unteren Abschnitts 8a sind die beiden Seitenplatten 8 über eine Frontplatte 11 fest verbunden, welche an den beiden Seitenplatten 8, stoff-, form- oder kraftschlüssig angeschossen ist. Vorzugsweise ist die Frontplatte 11 über deren beiden Höhenkanten hinweg an den beiden Seitenplatten 8 verschweißt. Grundsätzlich erfolgt die Verbindung der Frontplatte 11 an den beiden Seitenplatten 8 jedoch derart, dass die die beiden Seitenplatten 8 querbelastende Kräfte als Schubkräfte in die Frontplatte 11 einleitbar sind und dadurch die beiden Seitenplatten 8 in deren Querrichtung ausgesteift werden.

Gemäß der Fig. 1 sind die Haube 9 und vorzugsweise auch die Bodenplatte oder der Gerätesockel 6 an den Ober- und Unterkanten der beiden Seitenplatten 8 fixiert, vorzugsweise über die Kantenlänge hinweg angeschweißt, wobei auch die Frontplatte 11 an deren Unterkante mit der Bodenplatte 6 fest verbunden, vorzugsweise über deren Kantenlänge hinweg angeschweißt ist. Auf diese Weise wird durch die beiden Seitenplatten 8, die Haube 9, die Bodenplatte 6 und die Frontplatte 11 ein verwindungssteifes Gehäuse bereitgestellt, das an der Rückseite vorliegend vollständig und an der Vorderseite zumindest im Bereich des oberen Abschnitts 8b offen ist.

Wie aus der Fig. 1 in Verbindung mit den Fig. 2 und 3 zu entnehmen ist, sind an den beiden vorstehend genannten Öffnungen, welche durch den Zusammenbau der einzelnen Platten gebildet werden (also nicht in den Platten ausgeschnitten sind) Türen oder Klappen 2, 3 angeordnet. Diese sind vorliegend an jeweils einem Seitenteil (Seitenplatte) 8 schwenkbar anscharniert und weisen an ihren, den Scharnieren 4 gegenüberliegenden Höhenkanten Schließmechanismen 4a auf, welche mit entsprechenden Schlössern 4b an dem jeweils anderen Seitenteil (Seitenplatte) 8 in Eingriff bringbar sind. Die Türen/Klappen 2, 3 sind dabei (gemäß der Fig. 1) derart an dem jeweils einen Seitenteil 8 angeschlagen und mit dem jeweils anderen Seitenteil 8 verriegelbar, dass in Schließposition der jeweiligen Tür/Klappe 2, 3 diese schubbelastbar ist und damit zur weiteren Versteifung des Gehäuses 1 beiträgt, wie dies durch die in den Fig. 2 und 3 eingetragenen Kräftepfeile angezeigt wird. Alternativ zu den vorstehend genannten Schließmechanismen kann es natürlich auch vorgesehen sein, dass die Türen/Klappen 2, 3 an deren Längskante(n) in Schließposition mit dem jeweiligen Seitenteil 8 und optional mit der Haube 9 und/oder Bodenplatte 6 verschraubt oder verklemmt wird. Im Übrigen können die Türen 2, 3 mit einer randseitig umlaufenden Abkantung versehen sein, wodurch die Verwölbsteifigkeit der Türen 2, 3 erhöht wird. Diese Abkantung kann die beiden Seitenplatten 8, die Haube 9 und den Überhang 8c umgreifen und so eine stabilere Kopplung mit diesen Gehäuseteilen bewirken.

Schließlich ist aus der Fig. 2 entnehmbar, dass die beiden Seitenplatten 8 zumindest in deren oberen Abschnitten 8b (optional auch in den unteren Abschnitten 8a) mit im Wesentlichen horizontalen Schienen beschlagen sind, die als Auflager- und/oder Gleitschienen für Einlegeböden bzw. Auflageplatten 13 dienen. Auch können diese horizontalen Schienen so angeordnet sein, dass sie (mit) ein Auflager zum Sockel bilden. Gleichzeitig fungieren diese Auflager-/Gleitschienen als Aussteifung der Seitenplatten 8 zur Vermeidung von Verwölbungen im Fall einer äußeren Krafteinwirkung.

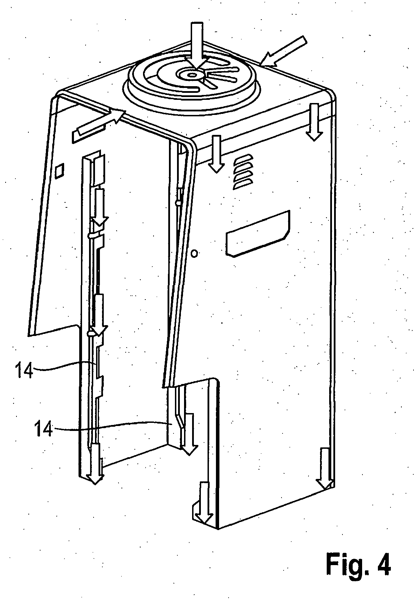

In der Fig. 4 ist das erfindungsgemäße Gehäuse 1 in teilweise montiertem Zustand abgebildet, wobei nur die beiden Seitenplatten 8 und die Haube 9 dargestellt sind, um einen besseren Einblick auf die Innenseiten der Seitenplatten 8 zu erhalten
Demzufolge weist die Haube 9, wie vorstehend bereits angedeutet wurde, den zentralen Anschlusssockel 10 vorliegend in Form eines Stutzens auf, an dem beispielsweise der Monitor 5 montierbar ist. Gleichzeitig bewirkt der Anschlusssockel 10 eine partielle Versteifung der Haube 9. Zudem ist die Haube 9 im vorliegende Beispiel an zwei sich gegenüberliegenden Rändern abgekantet, um flächig mit den Seitenplatten 8 in Anlage zu kommen. Dadurch kann die Verbindung der Haube 9 and den Seitenplatten 8 einfacher erfolgen und es können höhere Kräfte zwischen den Platten übertragen werden.

Schließlich weisen beide Seitenplatten 8 innenseitig tiefenbeabstandete, parallele Vertikalstreben 14 (ggf. alternativ oder zusätzlich zu den Horizontalschienen 12) auf, die ebenfalls zur Aufnahme/Auflagerung der Einlegeböden 13 vorgesehen sind, wofür die Vertikalstreben 14 Horizontalschlitze aufweisen können. Die Vertikalstreben 14 können vorzugsweise aus einem Hohlprofil (Trägerprofil) bestehen, innerhalb denen Kabel und Leitungen verlegt werden können. Dieses System ermöglicht somit auch eine getrennte Kabelverlegung nach Last- und Signalsträngen innerhalb der Vertikalstreben. Dadurch wird auch erreicht, dass die darin verlegten Kabel vom Strebenmaterial EMV - teilabgeschirmt werden.

In der Fig. 4 ist der Kraftverlauf in den Seitenplatten und der Haube durch Kraftpfeile angedeutet. Demnach bewirken die Vertikalstreben eine Verstärkung/Aussteifung der Seitenplatten zur erhöhten Aufnahme von Vertikalkräften etwa infolge des auf der Haube angebrachten Zusatzgeräts zur Verhinderung eines Ausbeulens der Seitenplatten. Die Haube 9 ist zur Aufnahme von Horizontalkräften (als Schubkräfte) vorgesehen, wobei der Anlenksockel 10 die Aufnahmefähigkeit der Haube 9 für Vertikalkräfte (Querkräfte) erhöht.

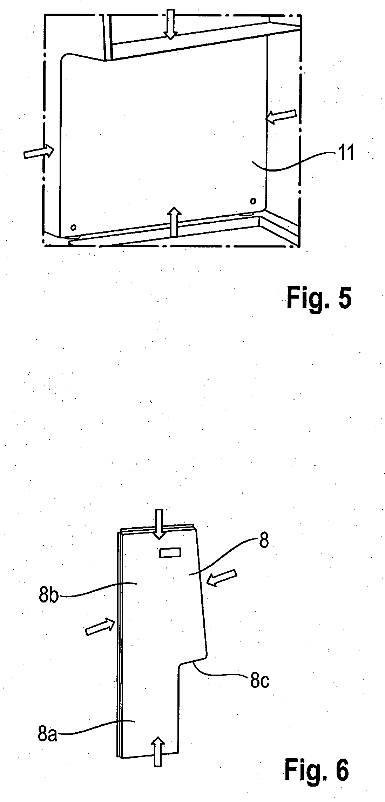

Zur besseren Verständlichkeit der aus einer herkömmlichen Handhabung der Dialysemaschine zu erwartenden Belastung des Gehäuses 1 sind in den Fig. 5 bis 9 die einzelnen Gehäuseplatten separat dargestellt und mit entsprechenden Kraftpfeilen versehen.

Demzufolge werden die beiden Seitenplatten 8 voraussichtlich im Wesentlichen mit horizontalen und vertikalen Schubkräften belastet, die teilweise auch Querkraftkomponenten enthalten können. Diese Querkraftkomponenten werden durch die horizontalen und/oder vertikalen Streben 12, 14 abgefangen und/oder über die Einlegeböden auf beide Seitenplatten 8 verteilt.

Die Haube 9 wird in erster Linie durch Horizontalkräfte belastet, kann aber auch infolge des integrierten Sockels 10 Vertikalkräfte aufnehmen und an die Seitenplatten 8 weitergeben.

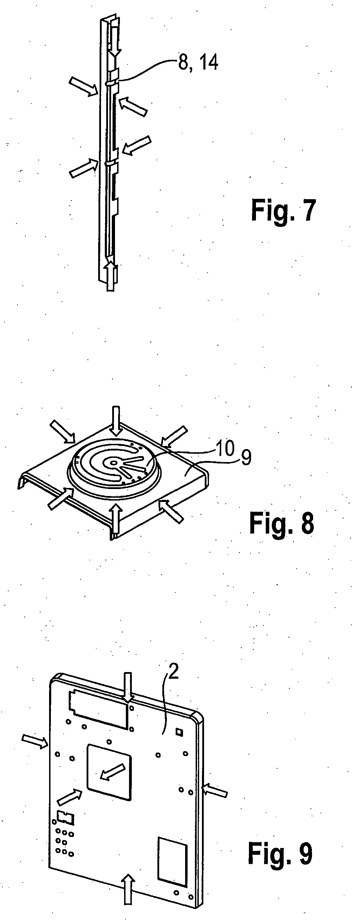

Die in der Fig. 9 gezeigte Vordertür 2 kann nur in geschlossenem Zustand zur Kraftweiterleitung dienen. In diesem Fall nimmt sie im Wesentlichen Vertikal- und Horizontalkräfte als Schubkräfte auf. Gleiches gilt für die Frontplatte 11 unterhalb der Vordertür 2, die im vorliegenden Ausführungsbeispiel gemäß der Fig. 5 keine innere Aussteifung hat und daher im Wesentlichen nur mit Horizontal- und Vertikalkräften als Schubkräfte belastbar ist. Dabei sei aber erwähnt, dass die Vordertür 2 auch mit internen Aussteifungen verstehen sein könnte und/oder eine entsprechende Profilierung haben könnten

Die vorliegende Erfindung betrifft zusammenfassend ein medizinisches Gerät zur extrakorporalen Blutbehandlung, das vorzugsweise als mobiles Gerät konzipiert ist. Erfindungsgemäß hat das Gerät ein Gerätegehäuse, das in selbsttragender Bauweise vorzugsweise vollständig rahmenlos ausgebildet ist.

## Patentansprüche

1. Medizinisches Gerät zur extrakorporalen Blutbehandlung mit einem Gerätegehäuse (1), in welchem zumindest hydraulische und elektronische Gerätebauteile eingehaust oder einhausbar sind, **dadurch gekennzeichnet, dass** das Gerätegehäuse (1) aus einer Mehrzahl von Schubplatten besteht, die zu einer selbsttragenden, rahmenlosen Gehäusekonstruktion zusammengefügt sind.

2. Medizinisches Gerät zur extrakorporalen Blutbehandlung gemäß Anspruch 1, **dadurch gekennzeichnet, dass** das Gerätegehäuse (1) aus einer Anzahl von stoff-, form - und/oder kraftschlüssig verbundenen Gehäuseplatten (6, 8, 9, 11) besteht, derart dass diese infolge äußerer, darauf einwirkender Kräfte gemäß einem normal zu erwartenden Gerätebetrieb im Wesentlichen nur schubbelastet sind.

3. Medizinisches Gerät zur extrakorporalen Blutbehandlung nach Anspruch 2, **dadurch gekennzeichnet, dass** die Gehäuseplattenverbindungen durch Verschweißen, Verlöten, Verkleben, Clinchen, Verstemmen, Verschrauben und/oder Vernieten bereitgestellt sind.

4. Medizinisches Gerät zur extrakoporalen Blutbehandlung gemäß Anspruch 2 oder 3, **dadurch gekennzeichnet, dass** die fest miteinander verbundenen Gehäuseplatten (6, 8, 9, 11) so angeordnet sind, dass das Gerätegehäuse (1) hierdurch an zumindest einer vorbestimmten Stelle zumindest einen Ausbruch oder eine Öffnung erhält.

5. Medizinisches Gerät zur extrakorporalen Blutbehandlung gemäß Anspruch 4, **dadurch gekennzeichnet, dass** die fest miteinander verbundenen Gehäuseplatten (6, 8, 9, 11) so zueinander angeordnet und ausgerichtet sind, dass trotz des zumindest einen Ausbruchs oder Öffnung der Kräfteverlauf oder die Kräfteweiterleitung durch die Gehäuseplatten (6, 8, 9, 11) nicht unterbrochen wird.

6. Medizinisches Gerät zur extrakorporalen Blutbehandlung gemäß Anspruch 4 oder 5, **dadurch gekennzeichnet, dass** die fest miteinander verbundenen Gehäuseplatten (6, 8, 9, 11) so zueinander ausgerichtet und platziert sind, dass das Grundprinzip der Schubbelastung der Gehäuseplatten im Wesentlichen aufrechterhalten bleibt.

7. Medizinisches Gerät zur extrakorporalen Blutbehandlung nach einem der Ansprüche 2 bis 6, **dadurch gekennzeichnet, dass** ausgewählte Gehäuseplatten (8, 9) partiell verstärkt sind, um so Anlenkstellen für Türen und/oder Klappen zu bilden und/oder um solche Abschnitte zu versteifen, die voraussichtlich mit Kräften unterschiedlich zur Schubbelastungsrichtung der betreffenden Gehäuseplatte beaufschlagt werden.

8. Medizinisches Gerät zur extrakorporalen Blutbehandlung nach Anspruch 7, **dadurch gekennzeichnet, dass** zumindest ausgewählte Verstärkungen (10, 12, 14) eine Gestalt/Struktur aufweisen, die sie zur Übernahme weiterer Funktionen, vorzugsweise die Funktion als Aufnahmegehäuse beispielsweise von Ventilatoren oder Lüftern, als Anschlusssockel für adaptive Zusatzgeräte (5) und/oder Auflagerplatten (13) und dergleichen, Funktionsträger geeignet machen.

9. Medizinisches Gerät zur extrakorporalen Blutbehandlung nach Anspruch 7 oder 8, **dadurch gekennzeichnet, dass** anscharnierte Türen und/oder Klappen (2, 3) mit dem Gehäuse (1) derart verriegelbar sind, dass sie hierdurch in den Kraftfluss im Gehäuse (1) eingebunden werden und so zur zusätzlichen Versteifung des Gehäuses im geschlossenen Zustand dienen.

10. Medizinisches Gerät zur extrakorporalen Blutbehandlung nach einem der vorstehenden Ansprüche, **dadurch gekennzeichnet, dass** das Gerät eine mobile Dialysemaschine mit integriertem Fahrwerk (7) ist.

11. Medizinisches Gerät zur extrakorporalen Blutbehandlung nach Anspruch 10, **dadurch gekennzeichnet, dass** das Fahrwerk (7) aus vorzugsweise vier Rollen besteht, von denen ausgewählte Rollen vertikal drehbar am Gehäuse (1) gehalten gelagert sind, wobei die Rollen an einer Gehäusebodenplatte (6) platziert sind.

## Claims

1. A medical device for extracorporeal blood treatment comprising a device housing (1) in which at least hydraulic and electronic device components are housed or can be housed, **characterized in that** the device housing (1) comprises a plurality of push cups, which are joined together such that a frameless housing of the self-supporting type is formed.

2. The medical device for extracorporeal blood treatment according to claim 1, **characterized in that** the device housing (1) consists of a number of housing plates (6, 8, 9, 11), which are connected to one another by a substance-to-substance bond, by positive locking engagement and/or by a force fit connection such that these housing plates will substantially only be acted upon by shear forces as a result of external forces acting thereon according to the normal device operation to be expected.

3. The medical device for extracorporeal blood treatment according to claim 2, **characterized in that** the housing plate connections are provided by welding, soldering, glueing, clinching, caulking, screw-fastening and/or riveting.

4. The medical device for extracorporeal blood treatment according to claim 2 or 3, **characterized in that** the fixedly interconnected housing plates (6, 8, 9, 11) are arranged such that at least one aperture or opening is defined at at least one predetermined location in the device housing (1).

5. The medical device for extracorporeal blood treatment according to claim 4, **characterized in that** the fixedly interconnected housing plates (6, 8, 9, 11) are arranged and oriented relative to one another such that, in spite of the at least one aperture or opening, the progression or transmission of forces through the housing plates (6, 8, 9, 11) will not be interrupted.

6. The medical device for extracorporeal blood treatment according to claim 4 or 5, **characterized in that** the fixedly interconnected housing plates (6, 8, 9, 11) are oriented and positioned relative to one another such that the basic principle of applying shear loads to the housing plates is substantially maintained.

7. The medical device for extracorporeal blood treatment according to one of the claims 2 to 6, **characterized in that** selected housing plates (8, 9) are partially reinforced so as to form articulation points for doors and/or flaps and/or so as to stiffen areas which will presumably have forces be applied thereto that differ from the shear load direction of the housing plate in question.

8. The medical device for extracorporeal blood treatment according to claim 7, **characterized in that** at least selected reinforcements (10, 12, 14) have a form/structure which makes them suitable for fulfilling additional functions, preferably the function as a housing for accommodating, e.g., fans or blowers, as a connection base for adaptive auxiliary devices (5) and/or supporting plates (13) and similar functional elements.

9. The medical device for extracorporeal blood treatment according to claim 7 or 8, **characterized in that** hinged doors and/or flaps (2, 3) are adapted to be locked to the housing (1) such that they will be incorporated in the distribution of forces in the housing (1) and will thus serve to additionally stiffen the housing in the closed condition.

10. The medical device for extracorporeal blood treatment according to one of the preceding claims, **characterized in that** the device is a mobile dialysis machine with an integrated carriage (7).

11. The medical device for extracorporeal blood treatment according to claim 10, **characterized in that** the carriage (7) comprises preferably four castors, among which selected castors are supported on the housing (1) such that they are vertically rotatable, the castors being placed on a base plate (6) of the housing.

## Revendications

1. Appareil médical servant au traitement du sang extracorporel, comprenant un boîtier d'appareil (1), dans lequel au moins des composants d'appareil hydrauliques et électroniques sont ou peuvent être logés, **caractérisé en ce que** le boîtier d'appareil (1) est constitué d'une pluralité de plaques de poussée, qui sont assemblées afin de former une structure de boîtier autoporteuse, sans bâti.

2. Appareil médical servant au traitement du sang extracorporel selon la revendication 1, **caractérisé en ce que** le boîtier d'appareil (1) est constitué d'un nombre donné de plaques de boîtier (6, 8, 9, 11) reliées par liaison de matière, par complémentarité de forme et/ou à force de telle manière que ces dernières sont exposées essentiellement seulement à une charge de poussée suite à des forces extérieures agissant sur elles selon un fonctionnement de l'appareil attendu dans des conditions normales.

3. Appareil médical servant au traitement du sang extracorporel selon la revendication 2, **caractérisé en ce que** les assemblages des plaques de boîtier sont réalisés par soudage, brasage, collage, clinchage, matage, vissage et/ou rivetage.

4. Appareil médical servant au traitement du sang extracorporel selon la revendication 2 ou 3, **caractérisé en ce que** les plaques de boîtier (6, 8, 9, 11) reliées les unes aux autres de manière solidaire sont disposées de telle manière que le boîtier d'appareil (1) présente de ce fait, au niveau au moins d'un emplacement préalablement déterminé, au moins un percement ou une ouverture.

5. Appareil médical servant au traitement du sang extracorporel selon la revendication 4, **caractérisé en ce que** les plaques de boîtier (6, 8, 9, 11) reliées les unes aux autres de manière solidaire sont disposées et orientées les unes par rapport aux autres de telle manière qu'en dépit du percement au moins au nombre de un ou de l'ouverture au moins au nombre de une, le parcours des forces ou le transfert des forces par les plaques de boîtier (6, 8, 9, 11) n'est pas interrompu.

6. Appareil médical servant au traitement du sang extracorporel selon la revendication 4 ou 5, **caractérisé en ce que** les plaques de boîtier (6, 8, 9, 11) reliées les unes aux autres de manière solidaire sont orientées et placées les unes par rapport aux autres de telle manière que le principe de base de la charge de poussée des plaques de boîtier reste essentiellement maintenu.

7. Appareil médical servant au traitement du sang extracorporel selon l'une quelconque des revendications précédentes 2 à 6, **caractérisé en ce que** des plaques de boîtier (8, 9) sélectionnées sont en partie renforcées afin de former ainsi des emplacements d'articulation pour des portes et/ou des trappes et/ou afin de raidir des segments de ce type, qui sont soumis de manière prévisionnelle à des forces différentes de la direction de la charge de poussée de la plaque de boîtier concernée.

8. Appareil médical servant au traitement du sang extracorporel selon la revendication 7, **caractérisé en ce qu'**au moins des renforcements (10, 12, 14) sélectionnés présentent une forme/une structure, lesquelles les rendent appropriés aux fins de la prise en charge d'autres fonctions, de préférence la fonction de boîtier de logement par exemple de ventilateurs, la fonction de socle de raccordement pour des appareils supplémentaires (5) adaptatifs et/ou pour des plaques d'appui (13) ou des supports fonctionnels similaires.

9. Appareil médical servant au traitement du sang extracorporel selon la revendication 7 ou 8, **caractérisé en ce que** des portes et/ou des trappes (2, 3) à charnières peuvent être verrouillées au boîtier (1) de telle manière qu'elles restent attachées de ce fait dans le flux de force dans le boîtier (1) et qu'elles servent ainsi, dans l'état fermé, au raidissement supplémentaire du boîtier.

10. Appareil médical servant au traitement du sang extracorporel selon l'une quelconque des revendications précédentes, **caractérisé en ce que** l'appareil est une machine de dialyse mobile comprenant un châssis (7) intégré.

11. Appareil médical servant au traitement du sang extracorporel selon la revendication 10, **caractérisé en ce que** le châssis (7) est constitué de préférence de quatre roulettes, parmi lesquelles des roulettes sélectionnées sont montées en étant maintenues au niveau du boîtier (1) de manière à pouvoir tourner verticalement, les roulettes étant placées au niveau d'une plaque de fond de boîtier (6).
